Europäisches Patentamt

European Patent Office

Office européen des brevets

(11) Veröffentlichungsnummer: **0 182 007**
**B1**

(12) **EUROPÄISCHE PATENTSCHRIFT**

(45) Veröffentlichungstag der Patentschrift: **17.10.90**

(21) Anmeldenummer: **85109688.3**

(22) Anmeldetag: **01.08.85**

(51) Int. Cl.⁵: **A 61 K 31/44,** A 61 K 9/48, A 61 K 47/00

(54) **Nifedipin enthaltende Darreichungsform und Verfahren zu ihrer Herstellung.**

(30) Priorität: **23.10.84 DE 3438830**

(43) Veröffentlichungstag der Anmeldung:
**28.05.86 Patentblatt 86/22**

(45) Bekanntmachung des Hinweises auf die
Patenterteilung:
**17.10.90 Patentblatt 90/42**

(84) Benannte Vertragsstaaten:
**AT BE CH DE FR GB IT LI LU NL SE**

(56) Entgegenhaltungen:
EP-A-0 004 650
EP-A-0 049 852
DE-A-2 614 864
LU-A- 65 929
US-A-4 442 112

CHEMICAL ABSTRACTS, Band 98, 1983, Zeite 383, Zusammenfassung nr. 221832y, Columbus, Ohio, US; KANEBO LTD: "Sustained-release nifedipine pharmaceuticals", & JP-A-58 46 019 (KANEBO LTD) 13. März 1983

(73) Patentinhaber: **Dr. Rentschler Arzneimittel GmbH & Co.**
**Postfach 320 Mittelstrasse 18**
**D-7958 Laupheim (DE)**

(72) Erfinder: **Köhne, Hans, Dr. Dipl.-Chem.**
**Rotbachweg 7**
**D-7959 Obersulmetingen (DE)**
Erfinder: **Lahr, Wolfgang**
**Silcherweg 29**
**D-7958 Laupheim (DE)**
Erfinder: **Schmersahl, Uwe Hein, Dr.**
**Friedhofweg 6**
**D-7930 Ehingen-Gamerschwang (DE)**

(74) Vertreter: **Dipl.-Ing. Schwabe, Dr. Dr. Sandmair, Dr. Marx**
**Stuntzstrasse 16**
**D-8000 München 80 (DE)**

(56) Entgegenhaltungen:

CHEMICAL ABSTRACTS, Band 98, 1983, Seite 368, Zusammenfassung 204351y, Columbus, Ohio, US; Wu, MINGFU et al.: "Preparation of nifedipine capsule and its clinical application", & YAOXUE TONGBAO 1983, 18(3), 139-40 (PHARM. LAB., YANAN PHARM: CO., SHAGAI, Peopl. Rep. CHINA)

Courier Press, Leamington Spa, England.

## Beschreibung

Die Erfindung betrifft eine neue, 1,4-Dihydro-2,6-dimethyl-4-(2-nitrophenyl)-3,5-pyridin-dicarbonsäre-dimethylester (Nifedipin) als Wirksubstanz enthaltende Darreichungsform, in der das Nifedipin molekulardispers in Form einer erstarrten Schmelze vorliegt, und zwar in einem Gemisch aus bei Raumtemperatur flüssigen und festen Polyethylenglykolen oder bei Raumtemperatur flüssigen, halbfesten und festen Polyethylenglykolen. Das wirkstoffhaltige Gemisch ist bei Raumtemperatur zähviskos bis fest und nicht mehr fließfähig und wird als Schmelze in Gelatine-Hart-Kapseln agefüllt und darin auf Raumtemperatur abgekühlt.

Der Wirkstoff 1,4-dihydro-2,6-dimethyl-4-(2-nitrophenyl)-3,5-pyridin-dicarbonsäuredimethylester (Nifedipin) gehört zur Gruppe der Calciumantagonisten und hat wegen seiner ausgeprägten antianginösen, antiarrhythmischen, antihypertensiven und cardioprotektiven Wirkung für die Langzeittherapie der koronaren Herzkrankheiten eine große Bedeutung erlangt, insbesondere beim lebensbedrohlichen Krisenbluthochdruck.

Aus der DE—OS 22 095 26 sind Gelatinebeißkapseln (Gelatine-Weich-Kapsel) bekannt, die mit einer Mischung aus einem Gewichtsteil Nifedipin, 650 Gewichtsteilen Polyalkylenglykolen mit einem Durchschnittsmolekulargewicht von 200 bis 4000, 1—10 Gewichtsteilen niederen Alkoholen und weiterer Formulierungshilfsmitteln gefüllt sind, wobei diese Gelatine-Weich-Kapseln neben einem Opakisierungsmittel einen Farbstoff enthalten, der Licht der Wellenlänge 250 nm—460 nm absorbiert.

Diese Darreichungsform und insbesondere das Verfahren zu ihrer Herstellung ist mit dem Nachteil behaftet, daß die Kapselherstellung und Kapselbefüllung in einem Arbeitsgang erfolgen muß, wozu neben einem großen technischen Aufwand in Form von speziellen Fertigungsmaschinen ein besonderes know how erforderlich ist. Naturgemäß handelt es sich deshalb bei dieser Gelatine-Weich-Kapsel um eine der teuersten festen Darreichungsformen. Nachteilig ist weiterhin, daß die Gelatine-Weich-Kapseln praktisch luftfrei abgefüllt werden müssen, damit ihre Stabilität gewährleistet ist. Daraus ergibt sich, daß das Füllvolumen bzw. Füllgewicht einer Kapselgröße nicht variierbar ist. Unterschiedliche Füllmengen erfordern unterschiedliche Kapselgrößen und damit unterschiedliche, teure Verpackungswerkzeuge. Gelatine-Weich-Kapseln sind auch in der US—A—4,442,112 beschrieben.

Die bekannte Gelatine-Weich-Kapsel stellt aufgrund der Zusammensetzung des Kapselmaterials und der Tatsache, daß die Kapsel eine völlig geschlossene Einheit bildet, außerdem an die Füllgüter besondere Anforderungen. So ist bei ihrer Herstellung unbedingt zu vermeiden, mit den Füllgütern eiweißfällende Stoffe in das Kapselinnere zu bringen, da diese die Gelatine zu hochmolekularen, wasserunlöslichen Produkten vernetzen. Nach der Vernetzung (Härtung) wird die Gelatine-Weich-Kapsel nämlich im Verdauungstrakt nur noch schwer oder überhaupt nicht mehr aufgelöst, so daß der darin enthaltene Arzneistoff verspätet oder gar nicht zur Resorption freigesetzt wird.

Zu den eiweißfällenden Stoffen gehören unter anderem Aldehyde. Polyalkylenglykole aber bilden während der Lagerung u.a. Aldehyde als Zersetzungsprodukte (Firmenbroschüre der Fa. Hoeschst AG, "Polyglykole Hoechst" 1981). Da nach dem Stand der Technik jedoch als Polyalkylenglykole, Polyethylenglykole zur Befüllung der Gelatine-Weich-Kapsel verwendet werden, muß auf den Einsatz von aldehydfreien Polyethylenglykolen besonders geachtet werden. Polyethylenglykole mit einem Gehalt von mehr als 5 ppm (5 Teile pro 1 Million Teile) Aldehyd können deshalb zur Herstellung derartiger Gelatine-Weich-Kapseln nicht mehr verwendet werden, um zu vermeiden, daß sich die Kapsel im Verdauungstrakt nicht mehr auflöst. Dies muß als entschiedener Nachteil der bekannten Darreichungsform angesehen werden.

Der Erfindung liegt die Aufgabe zugrunde, eine Nifedipin enthaltende Darreichungsform zur Verfügung zu stellen, die den darin enthaltenen Wirkstoff schnell freisetzt und dadurch rasch ansteigende, therapeutische wirksame Blutspiegel erzeugt, die ohne großen Aufwand hergestellt werden kann und trotz Verwendung von Polyethylenglykolen nicht die Gefahr in sich birgt, daß sich die Darreichungsform nicht mehr auflöst und den enthaltenden Wirkstoff nicht mehr freigibt.

Erfindungsgemäß wird diese Aufgabe derart gelöst, daß man das Nifedipin in Polyethylenglykol, vorzugsweise einer Mischung aus verschiedenen Polyethyleneglykolen in der Schmelze einarbeitet und diese Schmelze etwa 10°C oberhalb ihres Erstarrungspunktes in das Unterteil von Gelatine-Hart-Kapseln abfüllt, das Kapselunterteil mit dem Kapseloberteil verschließt, und somit die fertige Darreichungsform erhält.

Hartgelatine-Kapsen haben den entschiedenen Vorteil, daß sie als Leerkapseln handelsüblich sind und mittels üblicher Kapselfüllmaschinen, die durch ein zur Flüssigbefüllung geeignetes Abfüllorgan ergänzt sind, nach Belieben abgefüllt werden können, wobei die Füllmenge variabel ist und nach oben durch das Volumen des Kapselunterteils begrenzt ist. Gelatine-Hart-Kapseln haben darüber hinaus den Vorteil, daß sie aus zwei Teilen bestehen, die nach dem Befüllen ineinander geschoben werden. Dadurch ist kein hermetischer Verschluß beider Kapselteile gegeben.

Durch den zwischen Kapselober- und -unterteil bestehenden Spalt kann in jedem Fall Verdauungsflüssigkeit in das Kapselinnere dringen, selbst dann, wenn das Kapselfüllgut eiweißfällende Stoffe enthält, die eine Vernetzung der Gelatine bewirken könnten.

Nifedipin ist eine in Wasser praktisch unlösliche Substanz. Seine Löslichkeit in niedermolekularen, flüssigen Polyethylenglykolen hingegen ist gut. In höhermolekularen halbfesten und festen Polyethylenglykolen kann man das Nifedipin zwar in der Schmelze lösen, wobei jedoch nach Abkühlung auf Raumtemperatur das Nifedipin in den erstarrten Polyethylenglykolen wieder auskristallisiert. Zur schnellen Resorption innerhalb das Verdauungstraktes muß aber das Nifedipin dem Organismus in gelöster Form zugeführt werden, wobei die Lösungsgeschwindigkeit des Nifedipins der für die Resorption geschwindigkeitsbestimmende Schritt ist. Flüssige, niedermolekulare Polyethylenglykole wiederum lassen sich in Gelatine-Hart-Kapseln jedoch ohne Zusatz von Verdickungsmitteln nicht abfüllen. Es besteht die Gefahr des Auslaufens, der nur unter großen zusätzlichem technischen Aufwand durch Versiegeln der Kapselhälften (Banderolierung, Verklebung) begegnet werden kann. Verdickungsmittel hingegen können als Kristallkeime dienen und dazu führen, daß das gelöste Nifedipin aus der Polyethylenglykolmasse wieder rekristallisiert.

Es wurde nun überraschend gefunden, daß unter Verwendung von flüssigen, halbfesten und festen Polyethylenglykolen in bestimmten Mischungsverhältnissen sich abfüllfähige Massen herstellen lassen, in denen das Nifedipin molekular dispers vorliegt, die aber nach dem Abkühlen auf Raumtemperatur so viskos anfallen, daß das Auslaufen der Masse aus den Gelatine-Hart-Kapseln nicht mehr möglich ist und in der das Nifedipin nicht rekristallisiert.

Die Nifedipin enthaltenden Gelatine-Hart-Kapseln sind, dadurch gekennzeichnet, daß das Nifedipin in einem Gemisch aus bei Raumtemperatur flüssigen und halbfesten Polyethylenglykolen oder bei Raumtemperatur flüssigen und festen Polyethylenglykolen oder bei Raumtemperatur flüssigen und halbfesten und festen Polyethylenglykolen molekulardispers als erstarrte Schmelze voliegt, wobei das Verhältnis von flüssigen zu nichtflüssigen Polyethylenglykolen 7:23 bis 23:7 beträgt, der Erstarrungspunkt des Gemisches zwischen 25°C und 62°C liegt und das Gemisch Viskositäten von 0,1 bis 18000 Pas (1 bis 180000 Poise) aufweist, gemessen an einer Mischung aus bis zu 40 Gew.-% Wasser und Polyethylenglykolmischungen bei 20°C.

Erfindungswesentlich für die Zusammensetzung der abfüllfähigen Massen ist, daß im Sinne der vorstehenden Definition flüssige und nicht flüssige Polyethylenglykole im Verhältnis 7:23 bis 23:7, vorzugsweise 12:7 bis 20:12 verwendet werden, die durch einen Erstarrungspunkt zwischen 25°C und 62°C, vorzugsweise 35°C bis 45°C gekennzeichnet sind.

Unter flüssigen Polyethylenglykolen werden solche mit mittleren Molekulargewichten von 200 bis 600, halbfeste mit mittleren Molekulargewichten bis 1500 und feste Polyethylenglykole solche mit mittleren Molekulargewichten über 2000 verstanden (Katalog Pharmazeutische Hilfsstoffe, Vertrieb der Arbeitsgemeinschaft für pharmazeutische Verfahrenstechnik, 1974).

Ein weiteres Kennzeichen für die Zusammensetzung der erfindungsgemäß verwendeten Polyethylenglykolmischungen ist deren Viskosität (gemessen mittels Brookfield Rotationsviskosimeter, Typ RVT). Da die Mischungen bei Raumtemperatur nicht mehr fließfähig sein dürfen, ist deren Viskosität bei Raumtemperatur praktisch nicht meßbar. Es werden deshalb zweckmäßigerweise Mischungen mit bis zu 40 Gew.-% entmineralisiertem Wasser der Polyethylenglykolmischungen hergestellt, auf 20°C temperiert und mit vorgenanntem Gerät gemessen. Demnach besitzen erfindungsgemäß verwendbare Polyethylenglykolmischungen Viskositäten von 0,1 bis 18000 PaS (1 Poise bis 180 000 Poise).

So hat beispielsweise eine Mischung aus Polyethylenglykolen mit mittleren Molekulargewichten von 200 und 2000 im Verhältnis 22:8 als 80 Gew.-%ige wässrige Lösung eine Viskosität von 0,566 Pas (5,66) Poise und eine Mischung aus Polyethylglykolen mit mittleren Molekulargewichten von 200 und 35 000 im Verhältnis 8:22 als 60 Gew.-%ige wässrige Lösung eine Viskosität von 17200 PaS (172 000 Poise).

In die durch die Mischungsverhältnisse, den Erstarrungsbereich und die Viskosität gekennzeichneten Polyethylenglykolmischungen wird der Wirkstoff Nifedipin derart eingearbeitet, daß er in dem flüssigen Polyethylenglykol bei Raumtemperatur unter Rühren gelöst wird. Anschließend wird der nichtflüssige Anteil Polyethylenglykol zugegeben und die Mischung solange erwärmt, bis eine klare Schmelze entstanden ist, die ca. 10°C oberhalb ihres Erstarrungspunktes in Gelatine-Hart-Kapseln abgefüllt wird. Der Gehalt an Nifedipin in der Schmelze beträgt zwischen 1 und 10 Gew.-%, vorzugsweise zwischen 3 und 7 Gew.-% Der Schmelze können darüber hinaus lösungsvermittelnde und/oder die Viskosität erhöhende Hilfsstoffe zugesetzt werden, wie zum Beispiel Polyvinylpyrrolidone und/oder hochdisperse Kieselsäure, wobei die Auswahl so zu treffen ist, daß diese Hilfsstoffe die Rekristallisation nicht fördern. Selbstverständlich können weitere Hilfsstoffe verwendet werden, beispielsweise Substanzen, welche die Stabilität der an sich sehr lichtempfindlichen Substanz Nifedipin gegen die Zersetzung durch Lichteinfluß schützen, wie z.B. der Farbstoff "Gelborange S". Voraussetzung ist dabei wiederum, daß die Zusätze eine Rekristallisation des Wirkstoffes nicht begünstigen. Das Füllgewicht der Gelatine-Hart-Kapsel richtet sich nach der Konzentration des Wirkstoffes in der Schmelze und der gewünschten Dosis in der Darreichungsform. Die Größe der Gelatine-Hart-Kapsel richtet sich nach der Konzentration des Wirkstoffs in der Schmelze und der gewünschten Dosis in der Darreichungsform. Bevorzugt werden Kapseln, welche ein Volumen von 0,68 ml bis 0,21 ml aufnehmen können, insbesondere solche mit einem Volumen von 0,68 bis 0,3 ml, wie z.B. herkömmliche Gelatine-Hart-Steckkapseln der Größe Null, Eins, Zwei oder Drei. Der Gehalt an Nifedipin pro Kapsel beträgt 5 bis 20 mg, insbesondere 10 mg.

Die Herstellung der wirkstoffhaltigen Mischungen erfolgt dadurch, daß Nifedipin unter strengem Lichtausschluß im niedermolekularen, flüssigen Polyethylenglykol bei Raumtemperatur gelöst wird. Gegebenenfalls wird anschließend als Lichtschutz ein geeigneter Hilfsstoff, wie z.B. der Farbstoff "Gelborange S" eingerührt.

3

# EP 0 182 007 B1

Nach Zugabe der nichtflüssigen Polyethylengkykole wird die Mischung unter Rühren erwärmt, bis eine klare Schmelze erreicht wird.

Gegebenenfalls werden nun viskositätserhöhende und/oder lösungsvermittelnde weitere Hilfsstoffe eingerührt.

Die Mischung wird auf ca. 10°C oberhalb ihrer Erstarrungstemperatur abgekühlt und in Gelatine-Hart-Kapseln abgefüllt.

Es hat sich nun überraschende gezeigt, daß der Wirkstoff Nifedipin in der erfindungsgemäßen Darreichungsform über lange Zeit in molekulardisperser Form erhalten bleibt. Wider Erwarten wird der Wirkstoff in einem künstlichen Freisetzungsmodell nach USP XX innerhalb 30 Minuten zum überwiegenden Teil bis vollständig aus der Darreichungsform freigesetzt.

Vielmehr wäre zu erwarten gewesen, daß das Nifedipin im Kontakt mit dem wässrigen Freisetzungsmedium sofort rekristallisiert und damit praktisch unlöslich wird.

Da in vitro Versuche allein keine Aussage über die Verfügbarkeit des Wirkstoffes im menschlichen Organismus gestatten, wurde beispielhaft für den erfindungsgemäßen Gegenstand die Formulierung des Beispiels 6 an zehn gesunden Probanden auf Bioverfügbarkeit geprüft.

Als Vergleich diente sowohl eine echte Lösung von Nifedipin in reinem, flüssigen Polyethylenglykol mit einem mittleren Molekulargewicht von 200, als auch eine herkömmliche Tablette, in der das Nifedipin kristallin vorlag.

Die verabreichte Nifedipin-Dosis für die erfindungsgemäße Form und die Lösung betrug jeweils 10 mg, die für die Tablette 20 mg.

Die Dosis für die Tablette wurde deshalb doppelt so hoch gewählt, um sicher zu stellen, daß mit ausreichender Genauigkeit Plasmakonzentrationen gemessen werden können, da aufgrund der schlechten Löslichkeit des Nifedipin in wässrigen Systemen eine schlechtere Bioverfügbarkeit mit langsam ansteigender Plasmakonzentration zu erwarten war.

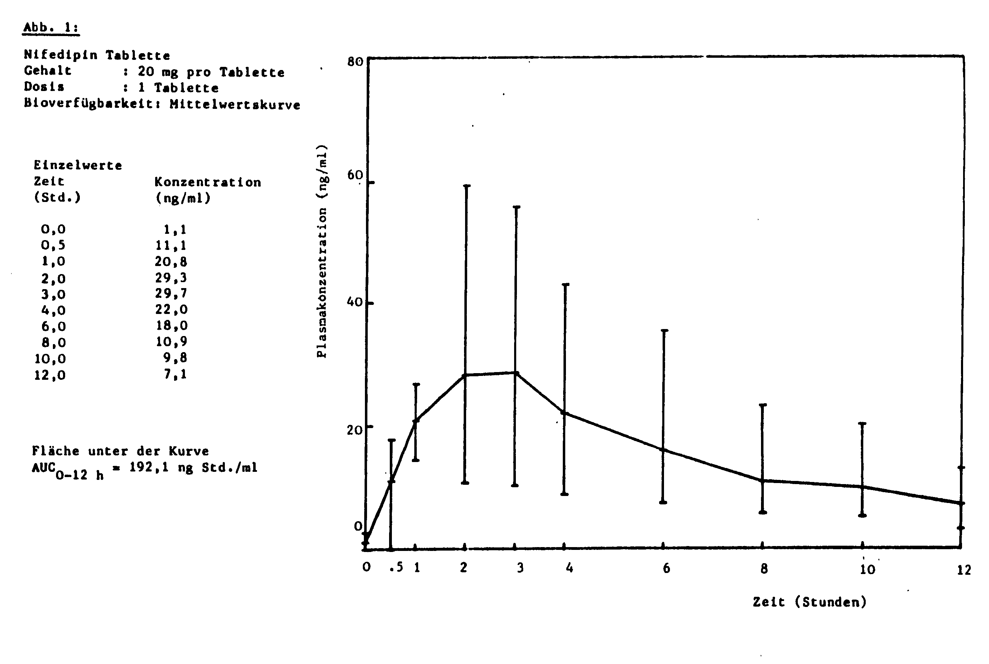

Die Lösung als direkter Vergleich zur erfindungsgemäßen Form wurde gewählt, weil zu erwarten war, daß mit dieser Form die beste Bioverfügbarkeit gegenüber einer festen Arzneiform erreicht wird. Die Überlegenheit von echten Lösungen gegenüber anderen Arzneiformen hinsichtlich der biologischen Verfügbarkeit zeigt beispielsweise Ritschel, Angewandte Biopharmazie, Stuttgart 1973, S. 346—347. Die Ergebnisse dieser Untersuchungen sind in Abb. 1—3 dargestellt.

Abb. 1 zeigt den zeitlichen Verlauf der Plasmakonzentration aus der herkömmlichen Tablette. Erwartungsgemäß ist der Anstieg der Plasmakonzentration wegen der niederen Lösungsgeschwindigkeit des Nifedipins in wässrigen Systemen langsam, die maximale Plasmakonzentration wird erst nach ca. 2 Stunden erreicht. Der Kurvenverlauf zeigt, daß die kristalline Wirksubstanz selbstretardierend wirkt.

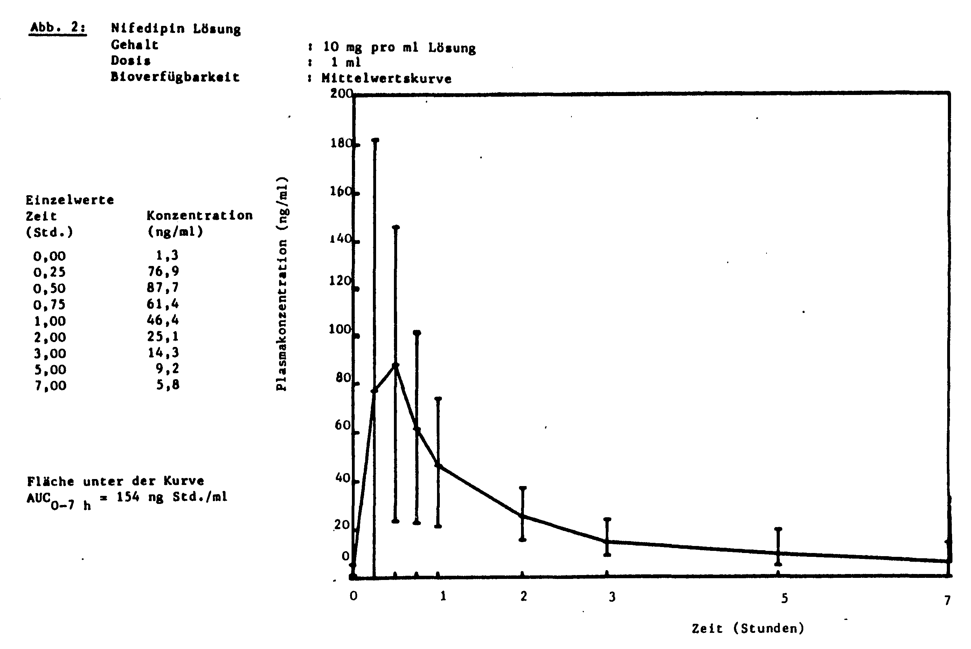

Abb. 2 zeigt den erwarteten zeitlichen Verlauf aus der Lösung, wobei die Plasmakonzentration bereits nach 30 Minuten auf einen max. Wert ansteigt, was damit zu erklären ist, daß gegenüber Beispiel 1 der die Resorption bestimmende Schritt, nämlich die Lösungsgeschwindigkeit des Wirkstoffs, entfällt.

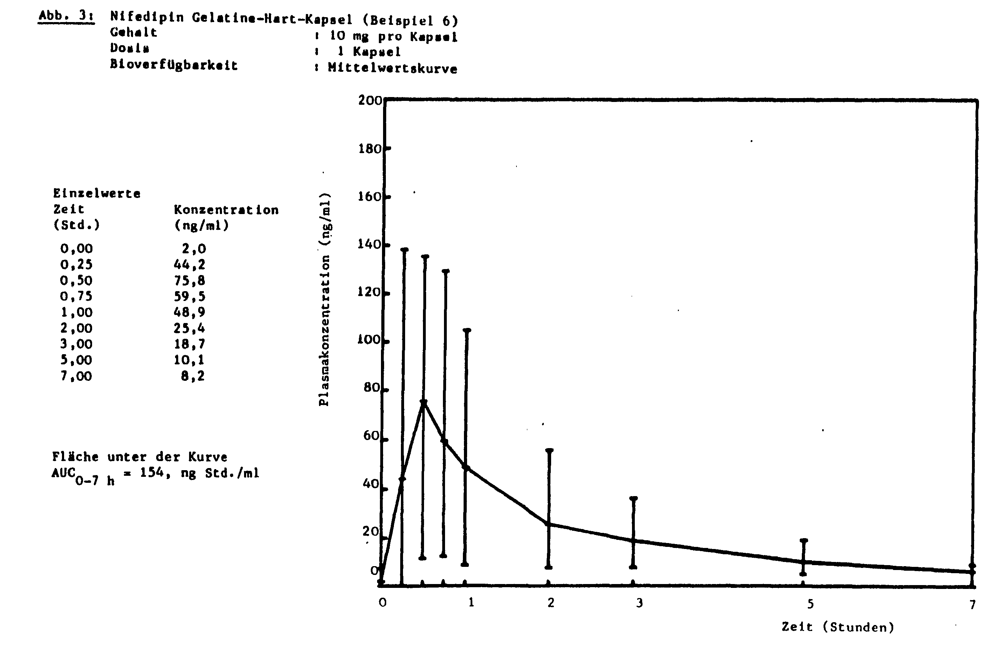

Überraschend waren jedoch die Ergebnisse aus Abb. 3, die zeigen, daß aus der erfindungsgemäßen Form annähernd gleich schnell wie bei dem Vergleich aus Abb. 2 hohe Plasmakonzentrationen erreicht werden; insbesondere unter dem Gesichtspunkt, daß für Nifedipin die untere Grenze für therapeutisch wirksame Blutplasmaspiegel bie 10 bis 15 Nanogramm pro Milliliter beträgt (Rämsch, Selecta 10, S. 860, vom 7. März 1983), obwohl die Darreichungsform, mit der erstarrten Schmelze zunächst gelöst werden muß. Der für die Resorption und die Resorptionsgeschwindigkeit bestimmtende Schritt, die Lösungsgeschwindigkeit des Kapselinhaltes, behindert das Ausmaß der Resorption offensichtlich nicht.

Vergleicht man darüber hinaus die Flächen unter der Plasmakonzentrations-Zeitkurve, AUC (Area under the curve), welche üblicherweise als Maß für die Bioverfügbarkeit dienen, so wird überraschend deutlich, daß die erfindungsgemäße Form gegenüber dem Vergleich 100% bioäquivalent ist.

| | |
|---|---|
| AUC für erfindungsgemäße Form (Abb. 3) | 154 ng Std/ml |
| AUC für Vergleich (Abb. 2) | 154 ng Std/ml |

Es war vielmehr zu erwarten gewesen, daß das Nifedipin im Verdauungstrakt während der Lösungsphase teilweise rekristallisiert, was zu einer gegenüber dem Vergleich verminderten Bioverfügbarkeit hätte führen müssen.

Demnach liegt mit dem erfindungsgemäßen Gegenstand eine Darreichungsform für Nifedipin vor, die gegenüber dem Stand der Technik unter wirtschaftlichen Vorteilen mit handelsüblichen Geräten hergestellt werden kann, die unempfindlich ist gegen die sich nachteilig auswirkende Gelatinehärtung durch aus Polyethylenglykolen sich abspaltende Aldehyde, die in der Dosierung je Kapselgröße einen weiten Spielraum bietet, eine exakte Dosierung des Wirkstoffes erlaubt und wegen der vollständigen Freisetzung des Wirkstoffes aus der Darreichungsform als Einzeldosis den gleichen therapeutischen Vorteil einer echten Lösung besitzt, wobei die rasche Anflutung des Wirkstoffes in therapeutisch wirksamen Konzentrationen im Blutplasma diese Form auch besonders zur Behandlung des Krisenhochdruckes geeignet macht.

An den Beispielen 1 bis 10 der nachstehenden Tabelle 1 wird die Erfindung erläutert.

4

# EP 0 182 007 B1

## TABELLE 1

Nachfolgende Beispiele sollen die Erfindung erläutern:

| Beispiel Zusammensetzung: (Gew. Teile) | 1 | 2 | 3 | 4 | 5 | 6 | 7 | 8 | 9 | 10 |
|---|---|---|---|---|---|---|---|---|---|---|
| Nifedipin | 5 | 5 | 5 | 5 | 5 | 5 | 5 | 10 | 5 | 5 |
| Polyethylenglykol Mittl. Mol. Gew. 200 | 70 | 100 | 80 | 100 | 80 | 60 | 90 | 110 | 35 | 80 |
| Polyethyleneglykol Mittl. Mol. Gew. 2000 | 80 | — | — | — | — | — | — | — | — | — |
| Polyethylenglykol Mittl. Mol. Gew. 6000 | — | 50 | — | — | — | 35 | 55 | 40 | 115 | 65 |
| Polyethylenglykol Mittl. Mol. Gew. 10.000 | — | — | 70 | — | — | — | — | — | — | — |
| Polyethylenglykol Mittl. Mol. Gew. 20.000 | — | — | — | 50 | — | — | — | — | — | — |
| Polyethylenglykol Mittl. Mol. Gew. 35.000 | — | — | — | — | 70 | — | — | — | — | — |
| Polyvinylpyrrolidon (Kollidon[R]25) | — | — | — | — | — | 5 | 4 | — | — | 5 |
| Gelborange S | — | — | — | — | — | — | 1 | — | — | — |
| Verhältnis Flüssiges PEG:nicht-flüssiges PEG | 14:16 | 20:10 | 16:14 | 20:10 | 16:14 | 12:7 | 18:11 | 22:8 | 7:23 | 16:13 |
| Gehalt Nifedipin in der Schmelze (Gew.-%) | 3,2 | 3,2 | 3,2 | 3,2 | 3,2 | 4,76 | 3,2 | 6,25 | 3,2 | 3,2 |
| **Viskosität (Poise)** | | | | | | | | | | |
| 80% Mischung 20% Wasser | 783 | — | — | — | — | — | — | 1.234 | — | — |
| 60% Mischung 40% Wasser | — | 6,600 | 5,4 | 6,7 | 988 | 1,7 | 2,1 | — | 6,1 | 2,7 |
| Erstarrungspunkt ca. °C | 34,5 | 38,0 | 40,0 | 40,0 | 43,5 | 36,5 | 37,5 | 37,5 | 41,0 | 37,5 |
| Kapselvolumen (ml) bzw. Kapselgröße | 0,3 3 | 0,3 3 | 0,3 3 | 0,3 3 | 0,3 3 | 0,3 3 | 0,68 0 | 0,5 1 | 0,3 3 | 0,3 3 |
| mg Nifedipin/Kapseln | 10 | 10 | 10 | 10 | 10 | 10 | 20 | 20 | 5 | 10 |
| in vitro Freisetzung in % (in 30 Min.) | 87,9 | 87,0 | 76,0 | 90,5 | 81,5 | 100,0 | 91,8 | 95,2 | 93,7 | 91,2 |

**Patentansprüche**

1. Nifedipin enthaltende Gelatine-Hart-Kapsel, dadurch gekennzeichnet, daß das Nifedipin in einem Gemisch aus bei Raumtemperatur flüssigen und halbfesten Polyethylenglykolen oder bei Raumtemperatur flüssigen und festen Polyethylenglykolen oder bei Raumtemperatur flüssigen und halbfesten und festen Polyethylenglykolen molekulardispers als erstarrte Schmelze vorliegt, wobei das Verhältnis von flüssigen zu nichtflüssigen Polyethylenglykolen 7:23 bis 23:7 beträgt, der Erstarrungspunkt des Gemisches zwischen 25°C und 62°C liegt und das Gemisch Viskositäten von 0,1 bis 18000 Pas (1 bis 180 000 Poise) aufweist, gemessen an einer Mischung aus bis zu 40 Gew.-% Wasser und Polyethylenglykolmischungen bei 20°C.

2. Nifedipin enthaltende Gelatine-Hart-Kapsel nach Anspruch 1, dadurch gekennzeichnet, daß das Verhältnis von flüssigen zu nichtflüssigen Polyethylenglykolen 12:7 bis 20:12 beträgt.

3. Nifedipin enthaltende Gelatine-Hart-Kapsel nach Anspruch 1, dadurch gekennzeichnet, daß der Erstarrungspunkt des Gemisches aus Polyethylenglykolen zwischen 25 und 62°C liegt.

4. Nifedipin enthaltende Gelatine-Hart-Kapsel nach Anspruch 1 bis 3, dadurch gekennzeichnet, daß der Gehalt an Nifedipin in der Mischung 1—10 Gew.-%, insbesondere 3—7 Gew.-% beträgt.

5. Nifedipin enthaltende Gelatine-Hart-Kapsel nach Anspruch 1 bis 4, dadurch gekennzeichnet, daß die wirkstoffhaltige Polyethylenglykolmischung zusätzlich viskositätserhöhende und/oder lösungsvermittelnde und/oder die Stabilität des Nifedipins begünstigende Zusätze enthält.

6. Nifedipin enthaltende Gelatine-Hart-Kapsel nach Anspruch 1 bis 5, dadurch gekennzeichnet, daß als viskositätserhöhende und/oder lösungsvermittelnde und/oder die Stabilität begünstigende Zusätze Polyvinylpyrrolidone und/oder der Farbstoff "Gelborange S" enthalten ist.

7. Verfahren zur Herstellung von Nifedipin enthaltende Gelatine-Hart-Kapseln nach Anspruch 1 bis 6, dadurch gekennzeichnet, daß das wirkstoffhaltige Polyethylenglykolgemisch als Schmelze in Gelatine-Hart-Kapseln abgefüllt wird, deren Füllvolumen zwischen 0,21 und 0,68 ml liegt.

**Revendications**

1. Capsule gélatineuse dure contenant de la nifédipine, caractérisée en ce que la nifédipine se présente en dispersion moléculaire dans un mélange de polyéthylèneglycols liquides et semi-solides à température ambiante ou de polyéthylèneglycols liquides et solides ou de polyéthylèneglycols liquides, semi-solides et solides à température ambiante sous forme de masse fondue solidifiée, le rapport entre les polyéthylèneglycols liquides et non-liquides étant de 7:23 à 23:7, le point de solidification du mélange étant compris entre 25°C et 62°C et le mélange présentant des viscosités de 0,1 à 18.000 Pa.s (1 à 180.000 poises), valeurs mesurées à 20°C pour un mélange contenant jusqu'à 40% en poids d'eau et des mélanges de polyéthylèneglycols.

2. Capsule gélatineuse dure contenant de la nifédipine selon la revendication 1, caractérisée en ce que le rapport entre les polyéthylèneglycols liquides et non-liquides est de 12:7 à 20:12.

3. Capsule gélatineuse dure contenant de la nifédipine selon la revendication 1, caractérisée en ce que le point de solidification du mélange de polyéthylèneglycols est compris entre 25 et 62°C.

4. Capsule gélatineuse dure contenant de la nifédipine selon les revendications 1 à 3, caractérisée en ce que la teneur du mélange en nifédipine est de 1 à 10% en poids, en particulier de 3 à 7% en poids.

5. Capsule gélatineuse dure contenant de la nifédipine selon les revendications 1 à 4, caractérisée en ce que le mélange de polyéthylèneglycol contenant la substance contient de plus des additifs augmentant la viscosité et/ou des agents de solubilité et/ou améliorant la stabilité de la nifédipine.

6. Capsule gélatineuse dure contenant de la nifédipine selon les revendications 1 à 5, caractérisée en ce que les additifs d'augmentation de viscosité et/ou de solubilité et/ou d'amélioration de stabilité contiennent des polyvinyl pyrrolidones et/ou le colorant "juane orange S".

7. Procédé pour la fabrication de capsules gélatineuses dures contenant de la nifédipine selon les revendications 1 à 6, caractérisé en ce que le mélange de polyéthylèneglycols contenant la substance active est introduit sous forme de masse fondue dans des capsules gélatineuses dures dont le volume de remplissage est compris entre 0,21 et 0,68 ml.

**Claims**

1. Nifedipine-containing hard gelatine capsule, characterized in that the nifedipine is present in molecular dispersion as a solidified melt in a mixture of polyethylene glycols which are liquid and semi-solid at room temperature or polyethylene glycols which are liquid and solid at room temperature or polyethylene glycols which are liquid, semi-solid and solid at room temperature, the ratio of liquid to non-liquid polyethylene glycols being from 7:23 to 23:7, the solidification point of the mixture being between 25°C and 62°C and the mixture having viscosities of 0.1 to 18,000 Pas (1 to 180,000 poise) measured on a mixture of up to 40% by weight of water and polyethylene glycol mixtures at 20°C.

2. Nifedipine-containing hard gelatine capsule according to Claim 1, characterized in that the ratio of liquid to non-liquid polyethylene glycols is from 12:7 to 20:12.

3. Nifedipine-containing hard gelatine capsule according to Claim 1, characterized in that the solidification point of the mixture of polyethylene glycols is between 25 and 62°C.

6

4. Nifedipine-containing hard gelatine capsule according to Claims 1 to 3, characterized in that the content of nifedipine in the mixture is 1—10% by weight in particular 3—7% by weight.

5. Nifedipine-containing hard gelatine capsule according to Claims 1 to 4, characterized in that the active compound-containing polyethylene glycol mixture additionally contains additives which increase the viscosity and/or facilitate dissolution and/or promote the stability of the nifedipine.

6. Nifedipine-containing hard gelatine capsule according to Claims 1 to 5, characterized in that polyvinylpyrrolidones and/or the dyestuff "Yellow orange S" is/are contained as additives which increase the viscosity and/or facilitate dissolution and/or promote the stability.

7. Method for the production of nifedipine-containing hard gelatine capsules according to Claims 1 to 6, characterized in that the active compound-containing polyethylene glycol mixture is poured as a melt into hard gelatine capsules whose filling volume is between 0.21 and 0.68 ml.

Abb. 1:

Nifedipin Tablette
Gehalt         : 20 mg pro Tablette
Dosis          : 1 Tablette
Bioverfügbarkeit: Mittelwertskurve

Einzelwerte

| Zeit (Std.) | Konzentration (ng/ml) |
|---|---|
| 0,0 | 1,1 |
| 0,5 | 11,1 |
| 1,0 | 20,8 |
| 2,0 | 29,3 |
| 3,0 | 29,7 |
| 4,0 | 22,0 |
| 6,0 | 18,0 |
| 8,0 | 10,9 |
| 10,0 | 9,8 |
| 12,0 | 7,1 |

Fläche unter der Kurve
$AUC_{0-12\ h}$ = 192,1 ng Std./ml

EP 0 182 007 B1

**Abb. 2:** Nifedipin Lösung

Gehalt : 10 mg pro ml Lösung

Dosis : 1 ml

Bioverfügbarkeit : Mittelwertskurve

Einzelwerte

| Zeit (Std.) | Konzentration (ng/ml) |
|---|---|
| 0,00 | 1,3 |
| 0,25 | 76,9 |
| 0,50 | 87,7 |
| 0,75 | 61,4 |
| 1,00 | 46,4 |
| 2,00 | 25,1 |
| 3,00 | 14,3 |
| 5,00 | 9,2 |
| 7,00 | 5,8 |

Fläche unter der Kurve

$AUC_{0-7 h}$ = 154 ng Std./ml

Plasmakonzentration (ng/ml) vs. Zeit (Stunden)

EP 0 182 007 B1

**Abb. 3:** Nifedipin Gelatine-Hart-Kapsel (Beispiel 6)
Gehalt : 10 mg pro Kapsel
Dosis : 1 Kapsel
Bioverfügbarkeit : Mittelwertskurve

Einzelwerte

| Zeit (Std.) | Konzentration (ng/ml) |
|---|---|
| 0,00 | 2,0 |
| 0,25 | 44,2 |
| 0,50 | 75,8 |
| 0,75 | 59,5 |
| 1,00 | 48,9 |
| 2,00 | 25,4 |
| 3,00 | 18,7 |
| 5,00 | 10,1 |
| 7,00 | 8,2 |

Fläche unter der Kurve
$AUC_{0-7\ h}$ = 154, ng Std./ml

Plasmakonzentration (ng/ml)

Zeit (Stunden)